# FASCICULE DE BREVET EUROPEEN

(11) **EP 1 525 016 B1**
(45) Date de publication et mention de la délivrance du brevet: **16.11.2011**
(21) Numéro de dépôt: 03753659.6
(22) Date de dépôt: 21.07.2003
(51) Int. Cl.: A61M 5/32, A61M 5/28

(54) **DISPOSITIF DE PROTECTION POUR UN ELEMENT INVASIF DU TYPE AIGUILLE**
SCHUTZVORRICHTUNG FÜR EIN INVASIVES ELEMENT IN FORM EINER NADEL
DEVICE FOR PROTECTING A NEEDLE-TYPE INVASIVE ELEMENT

(30) Priorité: 19.07.2002 FR 0209201
(43) Date de publication de la demande: 27.04.2005
(73) Titulaire: Biofront, 75014 Paris (FR)
(72) Inventeur: LAHLOU KHALID, Pierre, F-75014 Paris (FR)
(74) Mandataire: Texier, Christian
(86) Numéro de dépôt international: PCT/FR2003/002300
(87) Numéro de publication internationale: WO 2004/009149

(56) Documents cités:
- EP-A- 0 589 506
- EP-A- 0 963 763
- DE-A- 4 223 689
- US-A- 2 911 972
- US-A- 2 935 067
- US-A- 3 173 200
- US-A- 4 994 046
- US-A- 5 019 048
- US-A1- 2002 065 488

## Description

La présente invention concerne d'une façon générale les dispositifs d'injection médicale.

L'aiguille d'injection médicale est actuellement le moyen le plus utilisé pour ponctionner des fluides corporels et pour inoculer ou infuser des substances thérapeutiques dans les tissus des patients, de manière ponctuelle ou durable. Dans le cas d'une seringue, elle est couplée à un corps de pompe muni d'un piston et constitue le plus connu des dispositifs d'injection. L'accouplement entre le corps de pompe et l'aiguille est normalisé de longue date.

Le concept de la seringue a plus de cent ans et sa dernière révolution a été la transformation et la mise en oeuvre de matériaux plastiques en substitution au verre. Ceci a permis de généraliser l'usage de seringues jetables. Cependant, les seringues pré-remplies sur un marché en développement rapide ont peu bénéficié de cette évolution technique. Aujourd'hui les hautes performances de ce matériau assurent la fonction d'effet barrière jusque là réservée au verre.

Une nouvelle technologie de matière plastique à haute performance a déjà permis de préserver le contenu liquide injectable vis-à-vis de l'oxydation due aux infimes échanges gazeux, au travers des parois légèrement poreuses du contenant.

Il s'agit de la technologie du film multicouche. Son coût de mise en oeuvre est cependant très élevé.

Par ailleurs, si un usage unique de la seringue jetable est un progrès de l'hygiène, il n'est toutefois pas en soi une garantie face aux risques de contamination par le virus HIV ou autres, car bien que fabriquées pour être jetables, les seringues et aiguilles conservent toutes leurs fonctionnalités après le premier usage. Le ré-usage est ainsi une des sources de la propagation de l'épidémie du Sida dans certaines populations plus exposées ou à faible pouvoir d'achat.

La présente innovation a pour but d'apporter une amélioration de l'efficacité des dispositifs d'injection.

Pour ce qui concerne maintenant l'aiguille, moyen invasif indispensable à l'injection médicale, elle est connue pour son efficacité. Ainsi une injection médicale peut être sous-cutanée, intradermique, intramusculaire ou intraveineuse ou bien encore, l'aiguille peut être utilisée pour faire des prélèvements de fluides corporels ou des ponctions lombaires ou mammaires par exemple.

Dénigrée pour ses inconvénients comme l'appréhension de la douleur ou le risque de transcontamination, l'aiguille offre tout de même lé meilleur rapport coût/efficacité qui est lui-même la meilleure garantie de la pérennité de son usage, et on verra que l'invention n'est pas destinée à la remettre en cause. Ainsi, malgré l'émergence de nouveaux moyens d'inoculation sans aiguille, rares sont les vecteurs biologiques capables de véhiculer des éléments tels qu'une protéine, un peptide, un fragment de gène ou tout autre principe actif sans l'aide d'un instrument ou d'un dispositif d'injection permettant de franchir la barrière cutanée.

Toutefois, on verra également que rien ne s'oppose à la mise en oeuvre de l'invention avec de tels nouveaux vecteurs biologiques.

Toujours à propos de l'aiguille d'injection, les récentes techniques d'affûtage du biseau et l'usage unique, qui permettent de bénéficier du tranchant optimal, ont considérablement réduit la douleur de la piqûre, mais en même temps augmenté sa dangerosité.

It est aussi décrit un dispositif en vue d'améliorer la sécurité des praticiens en leur offrant une protection intégrée contre les risques de piqûre accidentelle qui peuvent être l'origine de contaminations très redoutées dans des milieux à forte charge virale.

A cet égard, les principales contraintes rencontrées pour améliorer la sécurité sont de nature économique et industrielle, outre bien entendu le respect de la bonne pratique opératoire impliquant de s'adapter aux gestes et habitudes professionnelles des agents de santé, et l'invention vise à satisfaire l'ensemble de ces contraintes.

En outre, l'invention vise à faire face par un même moyen à la diversité des aiguilles existant sur le marché. A cet égard, on verra que l'invention permet de s'affranchir de la limite bien connue du rapport coût/efficacité compte-tenu des investissements de lancement de nouveaux produits et l'épuisement des gisements de productivité du secteur, et de la résistance au changement des agents de santé concernant les gestes professionnels.

A ce sujet, la normalisation de l'accouplement aiguille/seringue a certes favorisé l'importance des séries et donc la baisse des coûts de production, mais il ne paraît pas exister de technique de sécurisation d'aiguille qui ne remette pas en cause ces dispositions normatives.

Sur le plan industriel, l'aiguille d'injection médicale est un consommable de très grande série. Son usage est estimé à mille unités par seconde dans le monde. C'est donc un marché de masse dont la barrière d'accès est particulièrement élevée, compte tenu de l'investissement nécessaire en machines dont les cadences de production peuvent atteindre 120 unités/seconde. UN autre objet encore de l'invention est de proposer un nouveau dispositif d'injection qui reste facilement industrialisable.

Un dispositif d'injection médicale selon l'invention pourra comme on l'a évoqué ci-dessus coopérer avec une aiguille d'injection, vendue en tant qu'accessoire autonome stérile, mono-usage, jetable, emballée individuellement pour être couplée, de façon connue en soi, par exemple à une seringue ou bien à un container sous vide pour permettre des ponctions en vue d'analyses ou encore à un système de perfusion ou d'infusion.

Le diamètre de l'aiguille et sa longueur sont normalisés en fonction la nature de l'acte médical. Son calibre (longueur, diamètre) et la longueur de son biseau LB sont assortis à chaque acte médical spécifique. L'unité de mesure internationale qui définit ce calibre est la Gauge selon le système AWG (American Wire Gauge), un code couleur universel ISO 6009 permettant une prise en compte visuelle et rapide du calibre ad hoc. Par exemple une aiguille d'injection intramusculaire de calibre « 21 G ½ » a une longueur de 39 à 40 mm environ et un diamètre de 0,80 ou 0,90 mm. Sa couleur distinctive est le vert pour le diamètre 0,80 mm et le jaune pour le diamètre 0,90 mm. Un pigment colorie dans la masse la matière plastique constituant le cône Luer femelle formant l'embase de l'aiguille. Ce cône Luer femelle forme l'embase d'accouplement aux dimensions normalisées avec le cône Luer mâle de tout type de seringue respectant la norme et plus généralement de tout dispositif de série normalisée IV. La canule est généralement enduite d'un film de silicone pour faciliter la pénétration dans les tissus.

L'accouplement se fait par coincement selon la loi de Morse, suivant un angle d'environ 6°. On décrira dans la suite, dans le cadre de fonctions annexes du dispositif de sécurité, comment ce coincement peut être renforcé pour dissuader quiconque de faire un second usage du dispositif d'injection.

Typiquement, un dispositif selon la présente innovation concernera plus particulièrement les aiguilles de calibre 19 à 27 Gauges et dont la longueur varie entre 12 et 30 mm. Cette collection regroupe la gamme d'aiguilles fines dont le diamètre est compris entre 0,40 et 0,60 à biseau court ou à biseau long, dédiées à l'usage hypodermique, et la gamme d'aiguilles dédiées aux prélèvements de fluides corporels à biseau court et dont le diamètre extérieur est supérieur ou égal à 1 mm.

La protection de la pointe invasive est essentielle au cours des différentes phases de l'utilisation de l'aiguille pour satisfaire à différents besoins de sécurité. Cette protection intégrée est devenue obligatoire selon la législation américaine depuis avril 2001, date d'entrée en vigueur de la loi dite « Needle Stick Prevention Act » promulguée en novembre 2000.

Dans le déroulement de l'acte opératoire, la pointe invasive de l'aiguille peut être altérée dès qu'elle est hors de son capot de protection. En cas d'altération il s'ensuit une vive douleur lors de la pénétration dans la peau du patient. Au cours de l'acte cette pointe peut provoquer une contamination accidentelle. Il peut en être de même si l'instrument est négligemment abandonné sur la voie publique par des usagers plutôt inconséquents ou inconscients.

Lors de la première phase, avant la piqûre, il est indispensable d'assurer que le tranchant n'est jamais en contact avec une partie dure qui pourrait un tant soit peu l'émousser, étant bien entendu que la forme, l'acuité de la pointe ainsi que le tranchant du biseau ont pour objectif commun de transpercer l'épiderme de la façon la moins douloureuse possible.

Lors de la deuxième phase, au cours de l'acte opératoire proprement dit, la pointe doit pouvoir être libérée pour permettre la pénétration selon des angles, des profondeurs et une visibilité spécifiques à la nature de l'acte prévu. Le dispositif de sécurité en position opératoire doit être stable et ne pas gêner l'exécution magistrale de l'acte opératoire.

Après l'injection ou le prélèvement (troisième phase), la prolifération des accidents de contamination par piqûre accidentelle impose que l'extrémité souillée soit protégée lors des diverses manipulations ; il en est de même si l'instrument est utilisé hors d'un contexte hospitalier.

Bien qu'il soit obligatoire de disposer, à bon escient, des containers spéciaux pour le recueil des aiguilles et seringues usagées, force est de constater que l'efficacité du dispositif de containérisation dépend de la discipline des agents de santé et non d'une loi physique propre aux contraintes mécaniques dévolues par le fabricant à la protection des personnes contre le risque de contamination. Il en va de même pour les seringues dites mono-usage, pré-remplies ou non, équipées d'une aiguille de première monte. Dans la mesure où l'usage de telles seringues se répand de plus en plus hors du contexte dispensaire, il est essentiel qu'une protection intégrée à l'aiguille et s'activant d'elle-même prévienne les piqûres accidentelles. Cette prévention est particulièrement nécessaire aux personnes non averties du danger et exposées par la nature de leur emploi ou de leur occupation à une transcontamination fortuite, comme des enfants jouant dans un bac à sable fréquenté à d'autres heures par des toxicomanes ou encore des cantonniers travaillant dans les bouches techniques de la voirie. Ces personnes, à leur insu, sont exposées à un risque mortel tant que le Sida ne sera pas vaincu.

La plupart des innovations passées en matière de dispositif de sécurité ont eu pour objectif de prévenir ce type d'accident à la source de l'approvisionnement en proposant aux fabricants la plus grande facilité possible de mise en conformité de leurs productions d'aiguilles avec les dispositions législatives en vigueur, si possible tout en satisfaisant au contraintes industrielles et commerciales du marché.

Ces dispositifs de sécurité ou de protection et leurs différents inconvénients vont être commentés ci-dessous.

On citera tout d'abord le dispositif de sécurité « sommaire » comme un capot ou un capuchon d'origine non intégré à l'aiguille, mais conçu comme un accessoire amovible rapporté. Il s'agit une pièce très peu technique, généralement produite industriellement par des fabricants spécialisés et vendue en vrac et au poids. Un capuchon rigide est conçu pour être retiré avant action et remis en place après l'acte opératoire, aux risques et périls de l'opérateur.

On citera ensuite les dispositifs de sécurité qui ont fait florès depuis le début des années 90. Ils ont pour point commun une inaptitude à couvrir la pluralité des besoins ou des pratiques, faute d'un principe de base permettant de couvrir une gamme complète d'aiguilles sécurisées.

Un exemple d'un tel dispositif connu est l'aiguille qui se rétracte à l'intérieur du corps de pompe de la seringue suivant l'action et la position du piston lors de l'aspiration ou de l'injection. L'enclenchement du débrayage au niveau de l'ancrage de l'aiguille dans l'extrémité de corps de pompe est actionné par différents mécanismes à ressort. Certains mécanismes sont actionnés manuellement, d'autres automatiquement. La réalisation suppose une nouvelle ingénierie de conception de l'outil industriel, et donc des investissements très coûteux qui contrarient la réponse au besoin universel de sécurité.

Dans une réalisation concrète connue commercialisée par Becton-Dickinson, un coulisseau cylindrique est monté en translation sur le corps de la seringue. Ceci impose toutefois une seringue spécifique et une production relativement coûteuse.

Un autre dispositif de sécurité comporte un bouclier qui coulisse en translation sur le corps de la canule ou encore une extension en forme de jambe articulée, qui implique dans l'un ou l'autre cas que la protection du biseau est assurée par une action consécutive à un geste volontaire de l'opérateur. Ce type de dispositif de protection concerne les injections intramusculaires dont la bonne pratique opératoire exige des gestes francs et énergiques permettant la pénétration en profondeur dans les tissus, sous un angle voisin de 90°. La réponse au besoin de sécurité est donc partielle.

Plus récemment, et dans certaines inventions sophistiquées, on a décomposé le geste sécuritaire en deux temps : dans un premier temps une pièce rapportée faisant effet ressort est enclenchée lors du dégagement de l'aiguille jusqu'à un cran d'arrêt ; dans un deuxième temps le cran d'arrêt est désactivé par un geste simple et précis provoquant la détente de l'effet ressort, suivie automatiquement par la protection programmée. La réponse au besoin de sécurité est ici limitée à un court segment de marché.

It est décrit un dispositif de sécurité intégré pouvant adopter au moins deux positions qui dépendent essentiellement des propriétés mécaniques de la matière à l'état de repos et sous la contrainte d'un geste simple de l'opérateur pour favoriser son adoption par les agents de santé sans nécessiter de grandes campagnes d'initiation ou de formation.

Un autre objet de l'invention est de préserver l'autonomie des fonctions de l'élément invasif d'une part, et d'accroître la cohérence de l'ensemble de ses fonctions : fonction intrusive, fonction manipulation, fonction de sécurité intégrée (non revendiquée), ceci sur la base d'une analyse précise de la gestuelle de l'opérateur, pour une part, et sur l'analyse économique permettant la constitution d'une gamme d'instruments d'injection devant satisfaire aux multiples besoins spécifiques de la bonne pratique opératoire, pour une autre part.

Concernant la sécurité des injections, il s'agit de contribuer à sa généralisation. Le but est d'offrir une possible synergie entre une technologie d'aiguille sécurisée, un mécanisme simplifié de propulsion de substances thérapeutiques, le tout susceptible de constituer nouvelle génération de dispositifs d'injection formant doses, individuelles emballées.

Une des fonctions non revendiquées du dispositif est, par la polyvalence d'une fonction d'ancrage d'un dispositif de protection d'aiguille, de couvrir également les besoins du marché traditionnel de l'aiguille, ainsi que des marchés futurs des nouveaux dispositifs d'injection à créer. Dans tous les cas, la sécurité des agents de santé sera rendue moins tributaire de la faible marge de manoeuvre des industriels de l'instrumentation médicale.

L'invention vise également à couvrir le marché des canules de première monte sur des instruments d'injection médicale, sans autre ingénierie de conception. En se fondant sur l'accouplement standard universel Luer mâle-femelle, l'ancrage pourra s'effectuer aussi bien en amont d'un cathéter court qu'en aval d'une seringue pré-remplie ou d'un instrument médical équipé d'une aiguille de première monte, telle qu'une seringue à insuline ou à tuberculine de faible diamètre. Pour les seringues de diamètre plus élevé, particulièrement pour les seringues pré-remplies en verre ou en matière plastique, l'objet des de proposer un dispositif de protection pouvant s'adapter à toute géométrie et à toutes dimensions, notamment en pouvant déplacer à souhait le lieu d'ancrage sur le dispositif d'injection, et en particulier vers l'amont d'un corps de pompe de seringue, sans gêner la lisibilité de la graduation.

Il en va de même pour les seringues à insuline dont les usagers souffrent généralement d'une baisse d'acuité visuelle liée au diabète : l'objet est ici que le dispositif de protection puisse exercer une fonction de loupe en améliorant la lisibilité de la graduation sur le corps de pompe, étant rappelé ici l'importance capitale de cette graduation pour le dosage de d'insuline.

La présente invention vise de seconde part à proposer un nouveau dispositif conteneur apte à coopérer avec une aiguille selon cet accouplement normalisé et à assurer simultanément au moins une fonction de conditionnement d'une substance injectable containérisation stérile et préservation à l'abri de l'air) et une fonction de propulsion de cette substance à travers l'aiguille.

L'invention vise également à proposer un dispositif permettant l'aspiration de fluides corporels par exemple pour analyse.

L'invention propose un dispositif tel que défini dans la revendication 1.

Des aspects préférés mais non limitatifs de ce dispositif sont définis dans les revendications dépendantes 2 à 7.

L'invention propose également un dispositif d'injection tel que défini dans la revendication 8.

Des aspects préférés de ce dispositif d'injection sont définis dans les revendications dépendantes 9 à 11.

D'autres aspects, buts et avantages de la présente invention apparaîtront mieux à la lecture de la description détaillée suivante d'une forme de réalisation préférée de celle-ci, donnée à titre d'exemple non limitatif et faire en référence aux dessins annexés, sur lesquels :
La figure 1A est une vue de dessus d'une aiguille munie d'une partie de protection d'un dispositif d'injection, la partie de protection étant dans une première position,
La figure 1B est une vue de côté d'une aiguille d'injection standard appartenant au dispositif d'injection,
La figure 1C est une vue de côté de l'aiguille et de la partie de protection, dans la position de la figure 1A,
La figure 1D est une vue de côté de l'aiguille et de la partie de protection, dans une seconde position,
La figure 2 est une vue de côté d'une aiguille d'injection standard et d'un embout standard de dispositif d'injection,
La figure 3 est une vue de côté d'un dispositif d'injection de type seringue et son aiguille, avec une partie de protection analogue à celle des figures 1A à 1D,
La figure 4A est une vue en perspective de profil d'une partie de retenue et d'expulsion d'un dispositif d'injection,
La figure 4B est une vue de face partielle de la partie de la figure 4A,
La figure 4C est une vue en coupe axiale de la partie des figures 4A et 4B, au repos,
La figure 4D est une vue de côté, à l'état comprimé, de la partie des figures 4A à 4C,
La figure 5 illustre graphiquement un contour préféré d'une zone compressible de la partie des figures 4A à 4D, et
Les figures 6 et 7 sont des vues en coupe axiale de deux variantes du dispositif de retenue et d'expulsion.

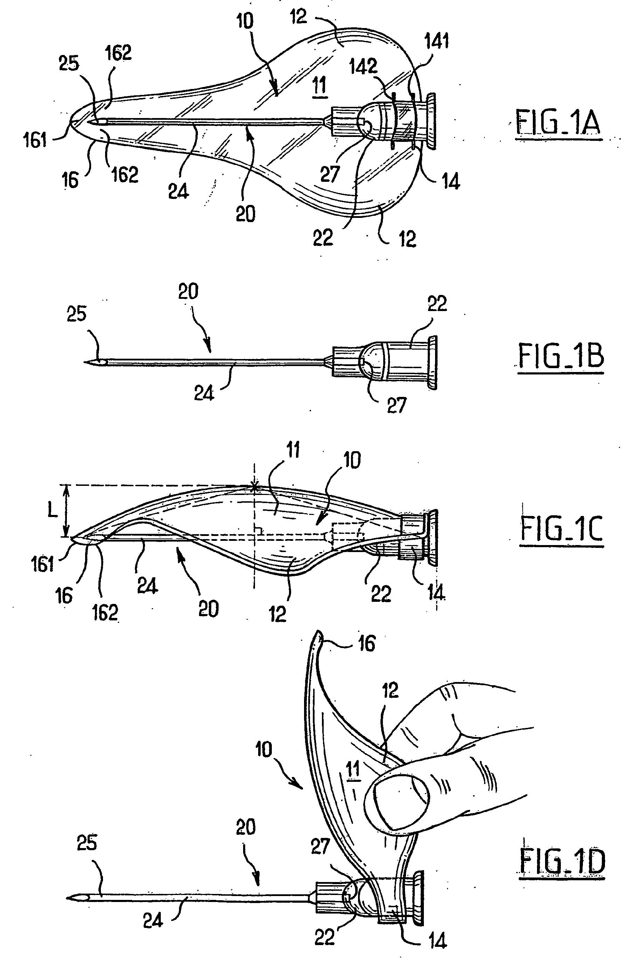

En référence tout d'abord aux figures 1A à 1D, on a représenté un dispositif de protection 10 ou carter associé ici à demeure à un cathéter court. Le carter peut également être fabriqué comme un semi-produit fini adaptable notamment à un cathéter court du commerce en lieu et place du capot de protection traditionnel de celui-ci, que cet élément soit fabriqué par le manufacturier d'origine pour être assemblé ensuite à son cathéter court ou à ses dispositifs d'injection équipés d'une aiguille de première monte, ou encore que cet élément soit fabriqué par un équipementier qui l'adapte aux produits originaux standards de ses clients ou fournisseurs fabricants.

Le carter 10 est une pièce monobloc en matière plastique thermo-souple transparente destiné à faire partie intégrante des dispositifs d'injection médicale, qu'il s'agisse de cathéter court, de seringues traditionnelles, y compris des seringues pré-remplies équipées d'une aiguille de première monte ou tout autre dispositif d'injection de série IV, en particulier un dispositif conteneur selon l'invention tel qu'il sera décrit plus loin.

La conception du carter 10 est telle que, premièrement, côté intrusif de l'article auquel il est destiné, soit le biseau acéré d'une l'aiguille, il assure la protection du tranchant du biseau, en toute circonstance, contre une agression mécanique du biseau avant le premier usage. Deuxièmement, après usage unique du dispositif, le carter, en position permanente de repos, occulte l'agressivité du biseau de façon passive pour éviter toute blessure, éraflure ou piqûre accidentelle imputable à la pointe souillée de l'aiguille. Troisièmement, dans sa conception côté aval de l'instrument médical auquel il est destiné, le carter possède des moyens pour la fixation sur le corps de l'instrument, par exemple le corps de pompe d'une seringue du commerce ou le corps de tout autre dispositif d'injection médicale, y compris des cathéters courts du commerce, assurant ainsi par son adaptabilité une plus large diffusion à toutes les gammes de dispositifs standards de série IV. Il s'agit d'un concept de produit doté d'un maximum de degrés de liberté pour favoriser la sécurité des injections en milieu hospitalier, lors de campagnes de vaccination et lors d'auto-injections hors milieu dispensaire.

Maintenant de façon plus détaillée, le carter 10 pour aiguille présente une forme générale inspirée de la forme d'une raie Manta dont les ailerons 12, 12 présentent un arrondi aux extrémités. La partie avant 16 du dispositif de sécurité constitue un bouclier qui vient épouser et circonscrire l'agressivité du biseau 25 de l'aiguille 24 sans s'y frotter, grâce aux forces élastiques internes du corps 11 de la pièce faisant fonction de carter, ce corps étant solidaire de deux éléments fonctionnels, à savoir la partie avant 16 ou bouclier » et une embase 14, le tout constituant une pièce monobloc réalisée par injection. La matière utilisée pour cette pièce 10 est par exemple une silicone translucide, telle que celle utilisée pour la fabrication de ventouses ou de tétines pour biberons.

En fonction de la longueur de la canule d'injection, les dimensions du carter 10 sont adaptées à la distance entre le bouclier et l'embase de la canule.

Par défaut, c'est-à-dire sans l'intervention volontaire de l'opérateur, le bouclier de protection 16 épouse mécaniquement le biseau 25. On mentionnera ici que d'après les études cliniques et les tests de terrain menés ces dernières années aux Etats-Unis, le caractère de protection automatique et indépendante de la volonté de l'opérateur est le mieux apprécié pour satisfaire aux besoins de sécurité sans modifier les gestes professionnels.

Comme on va le voir, ce dispositif de protection s'utilise avec des gestes simples, effectués par la seule main dominante de l'opérateur, en toute sécurité au cours de l'acte opératoire, sans qu'il n'y ait besoin de la coopération des deux mains pour accomplir l'intégralité de cet acte. Une fois les ailes 12, 12 soulevées symétriquement et pincées ensemble entre le pouce et l'index de l'opérateur, dans le plan vertical défini par la canule 24, le bouclier 16 se retrousse pour libérer la pointe 25 de l'aiguille 24.

La sécurité est ainsi assurée par défaut. La coordination entre les deux mains n'est pas nécessaire à l'accomplissement de l'acte opératoire, ce qui limite le risque de piqûre accidentelle. La main non dominante, celle plus généralement exposée aux accidents, reste toujours hors du rayon d'action de la pointe 25.

Ce dispositif de sécurité exploite exclusivement les propriétés physiques de la matière plastique du carter 10 : le fait de rebrousser symétriquement les ailerons 12 de l'horizontale à la verticale infléchit la courbure dudit carter qui passe d'une forme concave à une forme convexe avec franchissement d'un état d'instabilité.

La caractéristique mécanique, concernant le dispositif de protection, repose sur l'élasticité de la pièce 10. Ces forces sont dormantes quand la pièce 10 est au repos, comme une lentille concave. Ces forces de contrainte entrent en action sous l'impulsion imprimée par le geste naturel de l'opérateur quand il se saisit de l'instrument. L'opérateur retrousse les ailerons symétriques 12 du carter, et les pince fermement sensiblement dans le plan vertical passant par la canule 24. La canule est alors complètement dégagée, prête à inciser la peau et à pénétrer suivant l'angle que lui imprime l'opérateur. La manipulation est très précise et les mains de l'opérateur sont, grâce au carter 10, hors du champ d'action de la pointe de l'aiguille. La visibilité est en outre excellente.

On va décrire dans un exemple concret la liaison bouclier-embase pour un carter de sécurité destiné à équiper, en tant que partie intrusive, un cathéter court du commerce tel qu'une aiguille hypodermique standard de calibre 23 x 1, ce qui correspond à une canule de longueur utile 25 mm et de diamètre 0,60 mm.

Le carter de sécurité 10 coopère au niveau de son embase 14 avec le cône Luer femelle 22 du cathéter court 20.

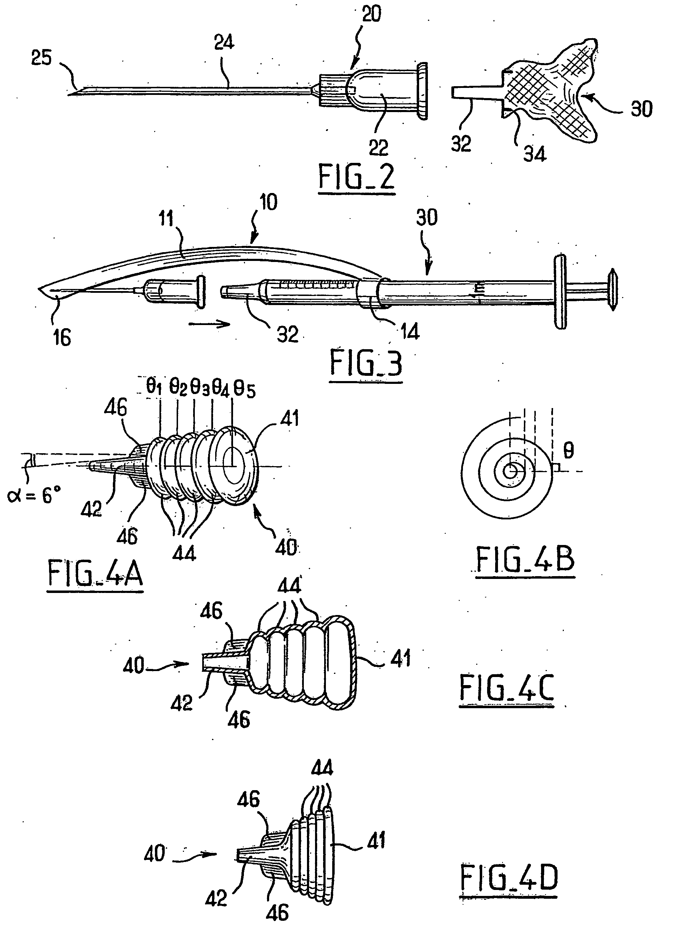

Dans d'autres réalisations comme les cas des aiguilles de première monte, une adaptation de l'embase du dispositif de sécurité par exemple au corps de pompe d'une seringue traditionnelle (voir figures 2 et 3) est réalisée pour satisfaire à certaines contraintes normatives telle que la transparence et la lisibilité obligatoire de la graduation sur ledit corps de pompe.

De façon plus particulière, le carter 10 est formé d'une pièce unique en matière plastique comme par exemple une silicone transparente, dont la forme géométrique est par exemple, au moins approximativement, générée par l'équation d'une spirale hyperbolique.

Le carter présente ainsi l'allure générale d'une lentille mince et souple, dont la longueur est légèrement supérieure à la longueur totale de l'aiguille 20 (canule + cône Luer) avec laquelle le carter 10 doit coopérer, et sa plus grande largeur est égale à environ trois quarts de sa longueur. Sa surface est lisse et antidérapante. L'épaisseur du carter peut être constante ou variable en fonction de la densité de la matière utilisée. Le carter comporte ses deux régions latérales 12 formant ailerons, dont les extrémités sont amincies jusqu'à la limite du périmètre de convexité fonctionnelle de la lentille souple. Cette disposition, détaillée plus loin, vise plus particulièrement une variante de position de livraison conforme aux contraintes d'emballage stérile du produit fini. Une autre des propriétés de la forme est de faciliter la fixation sur la peau du patient au niveau du dispositif 10 pour optimiser la stabilité dans le cas d'une fixation permanente.

Par exemple, le carter 10 présente en son centre géométrique une épaisseur qui est double de l'épaisseur au niveau de ses bords extérieurs 12. La variation d'épaisseur peut être constante ou par paliers.

La forme de la pièce par rapport au plan horizontal est concave, de sorte que la canule 24 se trouve disposée dans le plan horizontal de la forme concave comme la corde d'un arc, à l'intersection de ce plan horizontal avec le plan vertical défini par la canule.

Le carter monobloc 10 est naturellement arc-bouté sur la canule, sans contrainte du coté du biseau mais avec un ancrage ferme sur le cône Luer par son embase 14.

Le sommet de l'arc boutant se trouve approximativement à l'intersection de la perpendiculaire du milieu de la canule avec une ligne virtuelle dessinée sur le plan vertical défini par la canule. Cette ligne virtuelle forme un angle par exemple de 30° avec l'axe de la canule, valeur qui détermine la longueur L du segment qui relie le sommet du carter en position de repos au point médian de la canule. Il est ici observé que le point sommital du carter 10 peut, par conception, se situer en amont ou en aval du point médian de la canule sans modifier la fonctionnalité de la pièce. Tout au contraire la position de ce point devient un paramètre de conception pour optimiser la forme de la pièce en fonction de différentes contraintes.

Une simple pression de l'index de l'opérateur sur le sommet du carter provoque un effet de levier sur le bouclier 16, qui en se redressant dégage la pointe 25 de l'aiguille 24. Concomitamment les ailerons du carter se soulèvent spontanément de façon symétrique, suite à l'inversion de la courbure du carter, offrant alors une prise solide au pouce et à l'index de la main dominante de l'opérateur.

D'autres forces de contraintes de la matière du carter 10 peuvent entrer en action pour poursuivre le mouvement de redressement du bouclier et dégager complètement l'aiguille (voir figure 1D). Ces forces assurent une position stable du dispositif de sécurité pour les besoins de l'acte opératoire. Il s'agit notamment des forces antagonistes à la pression de la bride 14 qui forme l'embase du carter. Ces forces coopèrent à d'alignement du carter 10 à la verticale de l'aiguille.

L'embase 14 qui assure la fonction d'arrimage du dispositif de sécurité 10 est une échancrure ou lumière aménagée dans le corps de la pièce 10 et formée par exemple par de deux incisions parallèles 141, 142 en forme de trait de lame traversant de part en part toute l'épaisseur de la matière du carter, de sorte à dégager une bande de matière.

La forme de lanière est celle d'un parallélépipède rectangle, constituant une bride qui vient se serrer élastiquement sur le cône Luer 22 de l'aiguille. Le trait de lame amont 141 est positionné à une petite distance (typiquement 1 mm) du bord extérieur du carter 10 opposé au biseau 25. Le trait de lame aval 142 est positionné à par exemple 3 mm de distance du trait de lame amont 141. Les deux traits de lame parallèles forment un angle de 90° avec le grand axe du carter 10, de sorte à aménager un passage au corps du cône Luer tout en laissant la bride 14 totalement solidaire du carter par les petits cotés du parallélépipède rectangle.

La longueur de chaque trait de lame est voisine du diamètre extérieur du cône Luer 22, mesuré au niveau de la section conique droite située au niveau du trait de lame considéré. La surface interne de la lanière ou bride 14, en contact avec le matériau auquel elle s'arrime, en l'occurrence la surface externe du cône Luer, est une surface présentant, le cas échéant, des caractéristiques physiques qui augmentent la force d'adhésion entre les deux pièces sous la seule pression de l'élasticité de la matière mise en tension.

Le carter 10 est ainsi arrimé au cône Luer alors que le carter coiffe l'ensemble de l'aiguille 24.

Concernant le bouclier 16 protecteur du biseau 25, il est formé de trois éléments faisant partie intégrante de la matière du carter. Ces trois éléments sont disposés de telle sorte que la pointe de l'aiguille ne puisse ni transpercer le bouclier ni l'éviter.

Le premier élément est une digue frontale de matière 161 dans l'axe de la pointe de la canule. Les dimensions de cette partie (longueur, largeur, hauteur et épaisseur) sont suffisantes pour opposer une résistance sérieuse à l'agressivité de la pointe de la canule.

Les deux autres éléments 162 disposés symétriquement de part et d'autre de l'axe de la canule forment un canal ouvert dont les bords sont constitués par deux proéminences de matière en forme de demi-lune pour former un bec en V, avec la hauteur du V légèrement supérieure à la longueur du biseau 25 de la canule. L'écartement intérieur des proéminences de matière, à la base du V est ici d'environ 1,2 mm soit un écartement suffisant pour accueillir sans friction le diamètre maximal de canule siliconée, lequel diamètre n'excède pas ici 1,1 mm.

Le V est formé par les deux proéminences de matière 162 sur la face interne du carter, de sorte que la pointe du V se situe sensiblement dans l'alignement de l'axe de la canule 24 à une distance d'environ 1mm en amont du biseau et d'une distance d'environ 1mm + la longueur du biseau 25 + épaisseur de la digue 161 du bord extrême du carter. La fonction du bec en V est de caler la canule pour éviter que la matière du carter, toujours plus abrasive que l'acier tendre, ne subisse des mouvements de nature à émousser le biseau par frottement, matière contre matière, lors du transport des dispositifs d'injection équipés du dispositif de sécurité. Le bouclier 16 peut être renforcé par un dépôt notamment métallique pour accroître sa résistance à l'agressivité de la pointe de la canule. Dans ce cas, le dépôt est de préférence solidaire d'un tuteur (non représenté) allant du bec du bouclier 16 au sommet du carter 10 en position de protection pour opposer une résistance à la flexion en cas de choc frontal. En variante, cette résistance peut être assurée par une nervure injectée dans la matière du carter 10.

Une conception spécifique de la partie aiguille peut être envisagée pour assurer la fonction de percuteur visant à libérer la matière injectable située dans l'élément de contention et d'injection qui sera décrit plus loin, lors de l'accouplement entre l'aiguille et cet élément. A cet effet, comme on le verra, cet élément de contention pré-rempli est doté d'un bouchon ou opercule sous forme de membrane souple circulaire sertie par un procédé approprié à l'extrémité aval du cône Luer mâle de cet élément. Le diamètre de la membrane circulaire, dont le centre est coaxial aux deux dispositifs accouplés, est supérieur d'environ 0,8 mm au diamètre extérieur de l'extrémité du cône Luer mâle et inférieur de 0,2 mm au diamètre intérieur du cône Luer femelle 22 de l'aiguille. L'accouplement provoque une tension de la membrane et renforce le coincement Morse des organes mâle et femelle. La pénétration est limitée par les forces de coincement à environ 4 ou 5 mm. A mi-course la membrane tendue est exposée à un percuteur disposé à l'intérieur du cône Luer femelle de façon coaxiale aux deux dispositifs. Ce percuteur est en fait l'extrémité 27 de la canule opposée à sa pointe invasive, qui est de préférence taillée en biseau court. La canule 24 est sertie au cône Luer femelle 22 de telle sorte qu'en aval du sertissage ou collage jusqu'à l'extrémité du biseau intrusif 25, sa longueur utile corresponde à la norme. En amont, à l'intérieur du cône Luer femelle, sa longueur utile est calculée de telle sorte que l'extrémité du biseau court du percuteur rencontre la membrane de l'opercule de l'élément de contention sensiblement à mi-course de la pénétration consécutive à l'accouplement des deux dispositifs, soit environ 2 mm de l'embouchure du cône Luer femelle. Cette conception spécifique de l'élément de contention autorise que les dimensions extérieures du cône Luer, particulièrement sa longueur, soient aménagées pour offrir une meilleure prise facilitant l'assemblage des cônes mâle et femelle et la percussion de l'aiguille et de la membrane avec le minimum d'effort et de heurt.

Un dispositif de sécurité tel que décrit ci-dessus permet de faire co-exister les dispositifs d'injection traditionnels avec de nouveaux dispositifs d'injection basés sur la pénétration par aiguille.

L'autre partie du dispositif d'injection, formant élément de contention et d'expulsion de la substance thérapeutique à délivrer, se base sur un principe de cartouche individuelle mono-dose pré-remplie à usage unique. Dans la mesure où cet élément est détruit après un seul, unique et premier usage, certaines pratiques à la limite de la sécurité vaccinale tendront à disparaître. Pour accélérer l'éradication de ces pratiques ou du moins y contribuer, il est indispensable que le coût de fabrication d'un tel élément soit le plus bas possible, et en particulier inférieur ou égal au coût d'un usage par instrument traditionnel. Cette équation n'est certes pas réaliste dans les cas de ré-usage systématique programmé, mais l'arbitrage devient tout même plus favorable dans des économies de pénurie.

C'est dans le but de rendre ledit arbitrage le plus favorable possible que cette partie de l'invention a été mise au point, en faisant concourir l'ensemble des fonctions dispensaires à l'objectif de sécurité des injections médicales et particulièrement vaccinales. Les fonctions dispensaires dont il est question sont de trois natures différentes et complémentaires : fonctions intrusives, fonctions de conditionnement des substances thérapeutiques, et fonctions logistiques.

On a exposé plus haut la contribution des fonctions intrusives à la sécurité des injections.

Dans la suite, on va décrire des fonctions de conditionnement à savoir de la forme et des caractéristiques mécaniques propre à l'élément conteneur et, plus loin, à certains aspects logistiques qui concernent les capacités du dispositif d'injection objet de l'invention à satisfaire aux fonctions dispensaires telle que la stérilité, la disponibilité dans l'urgence et la chaîne du froid.

L'élément conteneur est ainsi une cartouche pré-remplie, mono-dose injectable dont l'extrémité aval est constituée d'un cône Luer mâle prévu pour épouser le cône Luer femelle du cathéter court spécifique 20 tel que décrit plus haut, cette cartouche étant operculée par une membrane fine et souple.

La forme géométrique des spires périphériques de cet élément est avantageusement générée par l'équation en coordonnées polaires d'une spirale logarithmique, paramétrée de telle sorte que le volume du contenant est égal de préférence à la dose prescrite pour les injections vaccinales soit par exemple 0,5 ml, 1 ml ou 1,5 ml.

On pense que les propriétés hydrodynamiques d'un tel contenant sont telles qu'à l'intérieur, un liquide sous pression s'évacue suivant les spires avec un effet de tourbillon.

Un tel agencement permet de faire l'économie d'une chambre de compression dotée d'un piston. Il est néanmoins souhaitable que les frottements entre les substances injectables et la surface des parois du contenant ne génèrent pas un différentiel de pression sensible entre le flux au centre du dispositif par rapport aux flux de la périphérie.

Pour réduire ces frottements, il est avantageux de modifier la forme galénique de la substance injectable et de son excipient liquide en ajoutant par exemple un polymère qui augmente la viscosité du liquide injectable au point de la transformer en gel. Les propriétés hydrophiles et hydrophobes du gel peuvent par ailleurs être mises à profit dans certaines applications thérapeutiques.

Il est également avantageux de recouvrir les parois internes du contenant, de préférence par une technique de plasma à froid, d'une couche de carbone amorphe, ceci étant particulièrement adapté si le contenant est fabriqué à base de polyéthylène-téréphtalate (PET) ou de polycarbonate cristal, dont les propriétés mécaniques de résistance à l'écrasement permettent une déformation régulière sous la pression du pouce de l'opérateur suivant l'axe orthogonal du dispositif induit par la forme en spirale logarithmique.

La déformation programmée de la matière constituante du contenant assujetti à la pression du pouce de l'opérateur s'effectue entre deux positions stables du dispositif d'injection, à savoir la position illustrée sur la 4A où les spires sont dilatées par la contrainte de la matière que la forme du moule imprime à l'empreinte lors de l'injection, et la position de la figure 4D où les spires sont écrasées suite à la déformation de la matière par la pression verticale du pouce de l'opérateur. La stabilité de la deuxième position dépend de l'épaisseur des parois du contenant, cette épaisseur étant calculée de sorte que la résilience de la matière ne provoque pas un mouvement de retour de nature à générer une aspiration post-opératoire.

Avantageusement, le contenant 40 est conçu pour qu'à pression constante du pouce de l'opérateur sur le culot arrière 41, l'écrasement progressif des spires soit sensiblement linéaire, avec un débit régulier et de préférence essentiellement constant.

Afin d'éviter la rotation du contenant autour de son axe lors de l'application de la pression du pouce, deux ailettes 46 disposées symétriquement dans la région de sortie du contenant, dans l'espace mort entre le cône Luer mâle 42 et la base du contenant, ont pour fonction de faciliter la prise de l'instrument entre l'index et le majeur de la main dominante de l'opérateur, de sorte que le culot du contenant subisse sans glisser sous le pouce une pression adéquate et adaptée à la résistance de l'injection, et que cette pression s'exerçant dans l'axe du dispositif soit bien répartie sur toute la surface du culot 41.

Afin d'adapter l'injection à la bonne pratique opératoire en matière médicale, plusieurs variantes de conception sont prévues. Ces variantes prennent en compte l'acquis médical sur la base de l'injection par seringues dotées d'une chambre de compression et d'un plongeur équipé ou non d'un piston en caoutchouc.

Notamment, l'étude de la dynamique des fluides sous pression lors de la réduction de diamètre entre le flux de liquide dans le cylindre de la seringue et le flux dans l'âme de l'aiguille a démontré qu'elle provoquait des turbulences, qui ont fait l'objet de modélisations mathématiques. Il s'ensuit une réduction à minima du diamètre intérieur des seringues (notamment pour l'injection d'insuline, de tuberculine, etc.) et une technologie de canule à paroi mince pour offrir, à diamètre extérieur égal, un diamètre intérieur maximal pour les canules. Les techniques de fabrication du piston en caoutchouc des seringues ont également été adaptées pour augmenter l'étanchéité tout en diminuant le frottement conte les parois du cylindre de la chambre de compression. Le liquide injectable est soumis lors de l'injection à une pression constante et linéaire sous l'effet de la translation du piston actionné par la pression du pouce de l'opérateur. La chambre de compression restant toujours cylindrique le liquide comprimé est chassé vers l'issue selon un diamètre constant.

Maintenant de retour à l'invention, l'effet d'entonnoir de la forme conique du contenant 40 induit une autre dynamique des fluides selon un modèle mathématique différent. Plus précisément, la réduction des frottements et l'augmentation de la viscosité de la substance injectable réduit les turbulences dans la mesure où la cavité du contenant est entièrement remplie lors du conditionnement automatique.

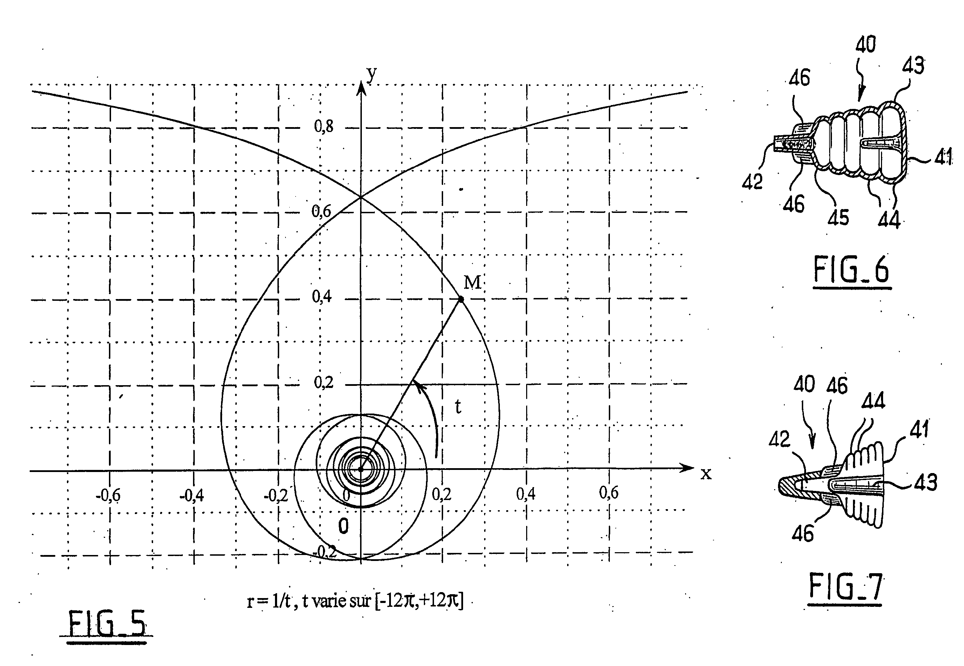

Concernant maintenant les injections intraveineuses, on sait que la moindre bulle d'air à l'intérieur du contenant interdira l'injection si cette bulle d'air ne peut être totalement évacuée. Maintenant en référence à la figure 6, on peut prévoir que la substance injectable soit sous forme de gel au maximum de sa viscosité, et que la dose injectable siège à l'intérieur du cône Luer mâle du 42 du contenant. Cette dose concentrée, de la taille par exemple d'un grain de blé, est destinée à être propulsée par un ergot 43 venu de matière avec le contenant, et qui épouse étroitement la cavité contenant cette dose. L'ergot 43 fait partie intégrante du culot et est positionné dans l'axe du dispositif. La pression du pouce de l'opérateur évacue l'air contenu dans le dispositif 40 par des évents latéraux 45 aménagés à la base du dispositif de sorte que le volume d'air évacué sous pression oppose une résistance faible ou nulle. En conséquence seule la résistance à la déformation de matière s'oppose à la pression du pouce de l'opérateur et la propulsion de la substance injectable se fait sous la poussée mécanique de l'ergot 43. Avantageusement, l'ergot 43 peut être dimensionné pour rester coincé dans le conduit d'expulsion après l'injection, pour garantir l'usage unique.

Une autre variante du contenant, conçue pour réaliser un prélèvement de fluide corporel, est représentée sur la figure 7 des dessins. Elle se distingue par les caractéristiques mécaniques du matériau, à savoir une matière plastique médicale thermo-souple. Le contenant comporte ici encore un ergot 43, mais qui a ici pour fonction d'obturer, en position comprimée, le cône Luer male, de manière à maintenir la chambre intérieure du contenant 40 en compression lors de l'accouplement de l'aiguille 20 sur le contenant avant l'intervention. Cet ergot 43 est conçu de telle sorte que l'étanchéité du dispositif soit assurée et éviter que l'air extérieur ne soit susceptible de pénétrer par la canule lors de l'opération d'accouplement. Ce contenant 40 destiné aux ponctions de fluides corporels est ainsi livré en position de compression. Le maintien de cette position stable est garanti par un capuchon étanche qui vient remplacer avantageusement la membrane formant opercule décrite précédemment.

On notera ici qu'une propriété particulière du PET est un déficit de conduction thermique par rapport au verre. Ce déficit de conduction est estimé à 25%.

Selon un autre aspect de l'invention, on prévoit le dépôt à l'intérieur de la cavité du contenant d'une couche de carbone amorphe. Ce dépôt, réalisé de préférence par traitement de surface, augmente l'étanchéité aux échanges gazeux périphérique. Il est également censé accroître le déficit de conduction thermique par rapport au verre, mais une telle propriété peut devenir tout à fait avantageuse dans les cas de non respect de la chaîne du froid, jusqu'à palier aux ruptures accidentelles ou programmées de cette chaîne du froid, particulièrement dans des régions du monde où l'équipement dispensaire laisse à désirer.

L'utilisation d'un revêtement interne de carbone amorphe présente d'autres avantages : tout d'abord, sa rigidité donne une meilleure résistance à l'écrasement du contenant, ce qui diminue le risque de décharge accidentelle ; en outre, la déformation du contenant effectuée pour l'injection cause une perte d'intégrité (rupture) de ce revêtement de carbone amorphe, et garantit la fonctionnalité d'usage unique en rendant le contenant inutilisable une seconde fois.

De retour au dispositif de protection décrit plus haut, on peut prévoir que le carter 10 soit réalisé en un matériau suffisamment malléable pour qu'au niveau de ses bords extérieurs, elle puisse être enroulée autour de la canule 20 et envelopper cette dernière avant que l'ensemble soit emballé en vue de la commercialisation, par exemple sous blister. Cette disposition permet de ne pas modifier sensiblement le processus automatiques existants de mise sous blister.

Enfin selon une autre variante, on peut prévoir de réaliser l'ensemble du dispositif d'injection (à l'exception de l'aiguille), à savoir le contenant et le dispositif de protection, d'un seul tenant, par exemple en silicone. Ceci est applicable notamment lorsque la durée de conservation du produit à injecter n'est pas critique.

Bien entendu, de nombreuses autres variantes et modifications pourront être apportées à l'invention, telle que définie dans les revendications.

## Revendications

1. Dispositif pour contenir un fluide, apte à coopérer avec un élément invasif tel qu'une aiguille notamment pour injecter un liquide thérapeutique ou pour prélever un fluide corporel, **caractérisé en ce qu'**il comprend une partie de montage (42) pour l'élément invasif, définissant un axe général du dispositif, et une enveloppe d'un seul tenant (40) déformable essentiellement selon ledit axe, ladite enveloppe étant de forme conique et comprenant une nervure périphérique en hélice (44) assurant la déformabilité privilégiée le long dudit axe général.

2. Dispositif selon la revendication 1, **caractérisé en ce que** la trajectoire de la nervure (44) répond, en projection dans un plan sensiblement perpendiculaire à l'axe général, à l'équation d'une spirale logarithmique.

3. Dispositif selon l'une des revendications 1 et 2, **caractérisé en ce que** la partie de montage (42) et l'enveloppe (40) sont réalisées d'un seul tenant.

4. Dispositif selon l'une des revendications 1 à 3, **caractérisé en ce que** l'enveloppe (40) est revêtue intérieurement de carbone amorphe.

5. Dispositif selon l'une des revendications 1 à 4, **caractérisé en ce qu'**il comprend dans la partie de montage un orifice de sortie de liquide pourvu d'un opercule apte à être perforé ou déplacé par l'extrémité intérieure d'une canule rapportée sur le dispositif.

6. Dispositif selon l'une des revendications 1 à 5, **caractérisé en ce qu'**il comprend en outre un ergot d'expulsion (43) prévu au niveau d'une face (41) de l'enveloppe opposée à la partie de montage et à pénétrer dans celle-ci pour expulser un fluide.

7. Dispositif selon la revendication 6, **caractérisé en ce que** le fluide est un gel visqueux.

8. Dispositif d'injection, **caractérisé en ce qu'**il comprend un dispositif conteneur selon l'une des revendications 1 à 7 et un élément invasif tel qu'une aiguille, ainsi qu'un dispositif de protection monté sur l'élément invasif.

9. Dispositif selon la revendication 8, **caractérisé en ce que** l'élément invasif et le dispositif conteneur sont assemblés par des cônes Luer standard.

10. Dispositif selon la revendication 8, **caractérisé en ce que** le dispositif conteneur et le dispositif de protection sont réalisés d'un seul tenant.

11. Dispositif selon la revendication 10, **caractérisé en ce que** le dispositif conteneur et le dispositif de protection sont réalisés en silicone.

## Claims

1. Device for containing a fluid, capable of cooperating with an invasive element such as a needle, in particular for injecting a therapeutic liquid or for sampling a body fluid, **characterized in that** it includes a mounting portion (42) for the invasive element, defining a general axis of the device, and a one-piece sheath (40) deformable essentially according to said axis, in which said sheath has a conical shape and includes a peripheral spiral rib (44) ensuring the preferred deformability along said general axis.

2. Device according to claim 1, **characterized in that** the path of the rib (44) satisfies, when projected in a plane substantially perpendicular to the general axis, a logarithmic spiral equation.

3. Device according to one of claims 1 and 2, **characterized in that** the mounting portion (42) and the sheath (40) are made in a single piece.

4. Device according to one of claims 1 to 3, **characterized in that** the sheath (40) is internally coated with amorphous carbon.

5. Device according to one of claims 1 to 4, **characterized in that** it comprises, in the mounting portion, a liquid outlet equipped with a closure capable of being perforated or moved by the internal end of a cannula attached to the device.

6. Device according to one of claims 1 to 5, **characterized in that** it also comprises an ejection tab (43) provided at a face (41) of the sheath opposite the mounting portion and intended to enter it in order to eject a fluid.

7. Device according to claim 6, **characterized in that** the fluid is a viscous gel.

8. Injection device, **characterized in that** it includes a container device according to one of claims 1 to 7 and an invasive element such as a needle, as well as a protection device mounted on the invasive element.

9. Device according to claim 8, **characterized in that** the invasive element and the container device are assembled by standard Luer cones.

10. Device according to claim 8, **characterized in that** the container device and the protection device are made in a single piece.

11. Device according to claim 10, **characterized in that** the container device and the protection device are made of silicone.

## Patentansprüche

1. Vorrichtung zum Enthalten eines Fluids, die geeignet ist, mit einem invasiven Element zusammenzuwirken, so wie einer Nadel, insbesondere zum injizieren einer therapeutischen Flüssigkeit oder zum Entnehmen einer Körperflüssigkeit, **dadurch gekennzeichnet, dass** sie einen Befestigungsteil (42) für das invasive Element, das eine allgemeine Achse der Vorrichtung definiert, und eine einstückige Hülle (40) umfasst, die im Wesentlichen entlang der Achse verformbar ist, wobei die Hülle eine konische Form aufweist und eine helixförmige Umfangsrippe (44) umfasst, die die bevorzugte Verformbarkeit entlang der allgemeinen Achse gewährleistet.

2. Vorrichtung nach Anspruch 1, **dadurch gekennzeichnet, dass** die Trajektorie der Rippe (44) in einer Projektion in eine Ebene, die etwa rechtwinklig zu der allgemeinen Achse ist, der Gleichung einer logarithmischen Spirale entspricht.

3. Vorrichtung nach einem der Ansprüche 1 oder 2, **dadurch gekennzeichnet, dass** der Befestigungsteil (42) und die Hülle (40) einstückig ausgeführt sind.

4. Vorrichtung nach einem der Ansprüche 1 bis 3, **dadurch gekennzeichnet, dass** die Hülle (40) innen mit amorphem Kohlenstoff beschichtet ist.

5. Vorrichtung nach einem der Ansprüche 1 bis 4, **dadurch gekennzeichnet, dass** sie in dem Befestigungsteil eine Flüssigkeitsausgangsöffnung umfasst, die mit einem Deckel versehen ist, der geeignet ist, durch eine innere Spitze einer Kanüle durchstochen oder verdrängt zu werden, die auf die Vorrichtung aufgesteckt ist.

6. Vorrichtung nach einem der Ansprüche 1 bis 5, **dadurch gekennzeichnet, dass** sie außerdem einen Austreibungsvorsprung (43) umfasst, der in Höhe einer Seite (41) der Hülle, die dem Befestigungsteil gegenüberliegt, und um in diese einzudringen, um ein Fluid auszustoßen, vorgesehen ist.

7. Vorrichtung nach Anspruch 6, **dadurch gekennzeichnet, dass** das Fluid ein viskoses Gel ist.

8. Injektionsvorrichtung, **dadurch gekennzeichnet, dass** sie eine Behältervorrichtung nach einem der Ansprüche 1 bis 7 und ein invasives Element, so wie eine Nadel, und auch eine Schutzvorrichtung umfasst, die auf dem invasiven Element befestigt ist.

9. Vorrichtung nach Anspruch 8, **dadurch gekennzeichnet, dass** das invasive Element und die Behältervorrichtung aus Kegeln gemäß Luerstandard zusammengesetzt sind.

10. Vorrichtung nach Anspruch 8, **dadurch gekennzeichnet, dass** die Behältervorrichtung und die Schutzvorrichtung einstückig ausgerührt sind.

11. Vorrichtung nach Anspruch 10, **dadurch gekennzeichnet, dass** die Behältervorrichtung und die Schutzvorrichtung aus Silikon ausgeführt sind.
